# EUROPÄISCHE PATENTSCHRIFT

(11) **EP 1 510 197 B1**
(45) Veröffentlichungstag und Bekanntmachung des Hinweises auf die Patenterteilung: **09.03.2016**
(21) Anmeldenummer: 04020183.2
(22) Anmeldetag: 25.08.2004
(51) Int. Cl.: A61K 6/00, A61K 6/093, C09D 183/04

(54) **Dentale Beschichtungsmaterialien**
Dental coating material
Matériau de revêtement dentaire

(30) Priorität: 29.08.2003 DE 10339912
(43) Veröffentlichungstag der Anmeldung: 02.03.2005
(73) Patentinhaber: Ivoclar Vivadent AG, 9494 Schaan (LI)
(72) Erfinder: Moszner, Norbert, Prof. Dr., 9492 Eschen (LI); Klapdohr, Simone, Dr., 6830 Rankweil (AT); Salz, Ulrich, Dr., 88131 Lindau (DE); Zimmermann, Jörg, 6890 Lustenau (AT); Rheinberger, Volker M., Dr., 9490 Vaduz (LI); Mühlhaupt, Rolf,Prof. Dr., 79117 Freiburg (DE)
(74) Vertreter: UEXKÜLL & STOLBERG

(56) Entgegenhaltungen:
- EP-A- 1 083 179
- EP-A- 1 197 539
- EP-A- 1 352 936
- WO-A-92/16183
- DE-A- 4 118 184
- DE-A- 19 746 708
- DE-A- 19 839 292
- DE-A- 19 903 177
- US-A1- 2003 114 554

## Beschreibung

Die vorliegende Erfindung betrifft polymerisierbare dentale Beschichtungsmaterialien, die beispielsweise die Entstehung von Plaque und die Bildung von Karies an dentalen Substraten, insbesondere an natürlichen Zähnen, verhindern können.

Die Beschichtung der Zahnhartsubstanz, vor allem des Zahnschmelzes und des Dentins, zum Schutz bei Zahnbehandlungen, als mechanischer Schutz vor Alterungsprozessen, aus kosmetischen Gründen, zur Therapie von Hypersensibilitäten oder zur Prävention von Karies, Gingivitis und Parodontitis ist bekannt. Zu diesen Zwecken werden im Stand der Technik unterschiedliche Materialien beschrieben.

Die EP 0 089 187 offenbart Schutzlacke, die neben Vinylacetat/Vinylchlorid Copolymer ein weiteres Copolymer enthalten, das neben Vinylacetat- und Vinylchlorideinheiten Carbonsäure- oder Carbonsäureanhydridgruppen aufweist. Die Copolymeren sind in organischem Lösungsmittel gelöst.

Die DE 37 17 762 offenbart ein Beschichtungsmaterial auf der Basis von Polystyrolharz/Kolophonium oder Kolophoniumderivaten, gelöst in einem organischen Lösungsmittel. Die Materialien sind zum Schutz von Zahnoberflächen vor einem unbeabsichtigten Ätzen vorgesehen.

Aus der US 6,083,421 sind Lacke auf der Basis von Carbamidperoxid, Filmbildner und Lösungsmittel zum Aufhellen von Zähnen bekannt.

Die WO 99/15131, EP 0 897 709, EP 1 216 681, EP 1 138 308, EP 0 716 845 und WO 02/26196 offenbaren Beschichtungsmaterialien, die neben filmbildendem Polymer und organischem Lösungsmittel Pigmente wie Titandioxid enthalten und hauptsächlich dazu dienen, die Zahnoberfläche mit einem weißen Überzug zu versehen.

Die US 5,133,957 offenbart adhäsive Schutzfilme zur Desensibilisierung von überempfindlichen Zähnen auf der Basis von Monomermischungen z.B. aus dem Reaktionsprodukt von N-Phenylglycin oder N-(p-Tolyl)glycin mit Glycidylmethacrylat und Monomeren, die durch die Reaktion von Anhydriden mit Hydroxyethyl-methacrylat zugänglich sind.

In US 5,330,746, US 5,403,577, US 5,139,768 und EP 0 381 445 werden Therapeutika zur Applikation auf die Zahnoberfläche beschrieben, die Strontium- oder Kaliumsalze in einem Trägerlack enthalten und sich zur Behandlung von Überempfindlichkeiten im Zahnhalsbereich eignen sollen.

In der DE 36 34 697 wird ein Lack zur Bekämpfung kariespathogener Keime wie *Streptokokkus mutans* und Lactobazillen beschrieben, der als Wirkstoffe Chlorhexidin-Digluconat und Thymol und/oder Carvacrol enthält.

Die US 4,883,534 und die EP 0 428 520 offenbaren Lacksysteme auf der Basis von Chlorhexidin. Darüber hinaus sind aus dem Stand der Technik Lacke mit anderen antibakteriellen Wirkstoffen wie Triclosan (2,4,4'-Trichloro-2'-hydroxydiphenylether) (WO 99/20227 und WO 98/48766), Cetylpyridiniumchlorid oder Benzalkoniumchlorid (EP 0 900 560) bekannt. Diese sind meist so formuliert, daß der Wirkstoff zusammen mit einem filmbildenden Polymer in einem organischen Lösungsmittel physikalisch gelöst ist.

Fluoridierungslacke zur Kariesprävention werden in US 3,969,499, DE 24 17 940 und DE 100 40 716 beschrieben.

Die US 4,324,630 und die WO 00/09030 offenbaren Schutzlacke, die auch ohne Fluor einen Säureangriff auf die Zahnhartsubstanz verhindern sollen.

Darüber hinaus sind Beschichtungsmaterialien auf der Basis hydrolytisch kondensierbarer und radikalisch polymerisierbarer Silane bekannt. Die hydrolytische Kondensation dieser Silane führt zu Polysiloxanen, die über polymerisierbare organische Gruppen thermisch, photochemisch oder redoxinitiiert gehärtet werden können (C. J. Brinker, G. W. Scherer, Sol-Gel-Science, Acad. Press, Boston etc. 1990, 839ff.). Hierbei werden anorganisch/organischen Netzwerke erhalten (H. Schmidt, Mat. Res. Soc. Symp. Proc. Vol. 32 (1984) 327-335; H. Schmidt, H. Wolter, J. Non-Cryst. Solids 121 (1990) 428-435).

Die EP 0 450 624 B1 offenbart polysiloxanhaltige Materialien, die sich besonders zur Beschichtung von Metallen, Kunststoffen, Papier, Keramik, Holz, Glas und Textilien eignen sollen. Die Polysiloxane werden durch Umsetzung von Silanen und ggf. weiteren hydrolytisch kondensierbaren Verbindungen, beispielsweise von B, Al, P, Sn und Pb, hergestellt. Die Beschichtungsmassen können darüber hinaus ungesättigte organische Verbindungen enthalten und je nach Wahl des Polymerisationsinitiators durch Licht oder thermisch gehärtet werden.

Ähnliche Polysiloxane, die sich als dentale Beschichtungsmassen eignen sollen, sind aus der DE 41 33 494 C2 bekannt.

In der DE 40 11 045 C2 und der EP 0 107 0499 A1 werden Lacke zur Beschichtung von Kunststoffsubstraten beschrieben, die neben einem Silan mit ethylenisch ungesättigten Gruppen ein zweites Silan mit einem Merkaptorest enthalten. Die Materialien sollen sich durch UV-Licht auch ohne die Zugabe eines Photoinitiators härten lassen.

Die DE 41 18 184 A1 offenbart Beschichtungszusammensetzungen auf der Basis von fluorhaltigen anorganischen Polykondensaten, die sich zur Beschichtung von Glas, Keramik, Metall, Kunststoff und Papier eignen und sich durch gute Antihafteigenschaften auszeichnen sollen. Die DE 195 44 763 A1 schlägt die Verwendung dieser Materialien zur Beschichtung von Zahnspangen und Zahnersatz vor.

Gemäß der DE 195 35 729 A1 soll die Beschichtung von Zahnersatz und Zähnen mit den Zusammensetzungen der DE 41 18 184 A1 diese vor einer Besiedelung ihrer Oberfläche durch Mikroorganismen schützen.

Aus der EP 0 171 493 B1 sind Beschichtungsmaterialien auf der Basis von anorganischen Polykondensaten aus löslichen Zirkonverbindungen und organofunktionellen Silanen bekannt, die eine kratzfeste Beschichtung von Kunststofflinsen und Kunststoffbrillengläsern erlauben sollen.

Die DE 38 36 815 A1 schlägt eine Verbesserung der aus der EP 0 171 493 bekannten Materialien durch die Zugabe von organischen Verbindungen vor, die funktionelle Gruppen aufweisen, welche erst im Laufe oder nach Beendigung der Härtung aktiviert werden, wie blockierten Polyisocyanaten und Polyestern.

Aus der EP 0 595 840 B1 und der EP 0 595 844 B1 sind Sol-Gel-Zusammensetzungen ausgehend von nicht radikalisch polymerisierbaren Alkoxiden, wie z.B. Tetraethoxysilan, Zirkoniumte-tra-sec.-butoxid oder Aluminiumtri-sec.-butoxid, bekannt, die sich nach hydrolytischer Kondensation zur Beschichtung von natürlichen Zähne eignen sollen. Die Härtung mit Laserstrahlen oder einer Gasflamme soll glasartige Überzüge ergeben.

Die WO 92/16183 offenbart Zusammensetzungen auf der Basis organisch modifizierter Kieselsäurepolykondensate, die sich für die Beschichtung von Zähnen und Zahnersatzteilen eignen sollen. Die ausgehärteten Überzüge sollen gegenüber der Plaqueanlagerung resistent sein.

Die WO 95/15740 und die US 6,312,668 betreffen oral anwendbare Beschichtungsmaterialien auf der Basis von polysiloxanmodifizierten organischen Polymeren. Diese werden dadurch erhalten, daß Silane mit polymerisierbaren Seitengruppen zusammen mit weiteren Komponenten einer radikalischen Polymerisation unterworfen werden. Die Silane können hydrolytisch kondensierbare Gruppen enthalten, die eine Kondensation der Polymere im Anschluß an die Polymerisation ermöglichen sollen. Die Endhärtung der Materialien erfolgt, gegebenenfalls nach Zugabe weiterer Monomere, durch erneute radikalische Polymerisation über polymerisierbare Reste, die in dem Polymer verblieben sind.

Die chemische Zusammensetzung und das Benetzungsverhalten der bekannten Beschichtungsmaterialien und der natürlichen Zahnhartsubstanz (Schmelz und Dentin) unterscheiden sich deutlich voneinander. Organisch modifizierte Polysiloxane weisen gewöhnlich hydrophobe Eigenschaften auf, während die Zahnhartsubstanz einen hydrophilen Charakter besitzt. Es ist zwar bekannt, daß hydrophobe Beschichtungsmaterialien im Hinblick auf die Verhinderung einer Besiedelung der Zahnoberfläche durch Mikroorganismen vorteilhaft sind, andererseits beeinträchtigen sie aber eine gleichmäßige Benetzung der Zahnoberfläche und die Haftung der Materialien, so daß eine Vorbehandlung der Zahnoberfläche mit Säuren und gegebenenfalls Haftvermittlern erforderlich wird. Die Säurebehandlung kann ihrerseits den Schutz nicht zu behandelnder Zähne mit geeigneten Schutzüberzügen notwendig machen, was den Gesamtaufwand der Behandlung zusätzlich erhöht.

Der Erfindung liegt die Aufgabe zu Grunde, Beschichtungsmaterialien bereitzustellen, die sich besonders für dentale Zwecke eignen und die ein gutes Benetzungsverhalten sowie eine hohe Selbsthaftung, insbesondere auf der Oberfläche von künstlichen und natürlichen Zähnen, zeigen, gleichzeitig jedoch die Anlagerung von Mikroorganismen, insbesondere von Plaque, und anderen unerwünschten Komponenten wirksam unterdrücken.

Erfindungsgemäß wird diese Aufgabe durch polymerisierbare , dentale Beschichtungsmaterialien gelöst, die
(1) 1 bis 80 Gew.-%, vorzugsweise 20 bis 60 Gew.-% und besonders bevorzugte 30 bis 50 Gew.-% mindestens eines Polykondensats auf der Basis von
   (A) einem oder mehreren radikalisch polymerisierbaren Silanen der Formel (I)

      (R¹ₐ-R²)ₘR³ₙSiX₍₄₋ₘ₋ₙ₎ (I),

      in der R¹ eine radikalisch polymerisierbarer Gruppe ist, R² entfällt oder ein zwei- (a=1) oder dreiwertiger (a=2) substituierter oder unsubstituierter Kohlenwasserstoffrest mit 1 bis 30 Kohlenstoffatomen ist, wobei die Kohlenstoffketten des Kohlenwasserstoffrestes durch 1 oder mehrere, vorzugsweise 1 bis 5, Heteroatome, insbesondere 0 und/oder S, 1 oder mehrere, vorzugsweise 1 bis 5, Carbonsäureestergruppen (-CO-O- oder -O-CO-), Carbonsäureamidgruppen (-CO-NR⁷- oder -NR⁷-CO- mit R⁷ = H oder C₁-C₅-Alkyl), Urethangruppen (-HN-CO-O- oder -O-CO-NH-) und/oder Aminogruppen (-NR⁸- mit R⁸ = H oder C₁-C₅-Alkyl) unterbrochen sein können, R³ ein C₁-C₁₂-Alkyl- oder C₃-C₁₂- Cycloalkylrest, ein C₆-C₁₂-Arylrest, C₆-C₁₂-Arylalkyl oder C₆-C₁₂-Alkylaryl ist, X Halogen oder ein C₁-C₃-Alkoxyrest ist, a 1 oder 2 ist, m eine ganze Zahl von 1 bis 3 ist und n eine ganze Zahl von 0 bis 2 ist, wobei die Summe von m und n nicht größer als 3 ist, und gegebenenfalls
   (B) einem oder mehreren Silanen gemäß der Formel (II)

      R⁴₍₄₋ₚ₎SiYₚ (II),

      in der R⁴ ein C₁-C₁₂-Alkylrest oder Phenylrest ist, der jeweils durch -NH₂, -SH oder -F substituiert sein kann, Y Halogen oder ein C₁-C₃-Alkoxyrest ist, und p eine ganze Zahl von 1 bis 4 ist, und/oder
   (C) einer oder mehreren Metallverbindungen der Formeln (III) und/oder (IV)

      AlZ₃ (III)

      Me(O)ᵣZₛ (IV),

      in denen Z Halogen oder ein C₁-C₆-Alkoxyrest ist, Me Zirkonium oder Titan ist, r 0 oder 1 ist, s eine ganze Zahl von 1 bis 4 ist, wobei die Summe der Wertigkeiten der Reste O und Z der Wertigkeit des Metalls entspricht,
(2) 0,1 bis 5 Gew.-%, vorzugsweise 0,2 bis 3 Gew.-% und besonders bevorzugt 0,5 bis 2 Gew.-% eines oder mehrerer Initiatoren für die radikalische Polymerisation und
(3) 0 bis 60 Gew.-%, vorzugsweise 20 bis 55 Gew.-%, besonders bevorzugte 30 bis 50 Gew.-% organisches, radikalisch polymerisierbares Monomer
   enthalten, dadurch gekennzeichnet, daß sie außerdem
(4) 1 bis 50 Gew.-%, vorzugsweise 5 bis 30 Gew.-%, besonders bevorzugt 10 bis 20 Gew.-% eines oder mehrerer radikalisch polymerisierbarer Monomere der Formel (V),

   R⁵ₜ-Sp-A_{w} (V),

   in der R⁵ eine radikalisch polymerisierbare Gruppe ist, t eine ganze Zahl von 1 bis 5 ist, Sp ein Kohlenwasserstoffrest mit 1 bis 30 Kohlenstoffatomen ist, wobei die Kohlenstoffketten des Kohlenwasserstoffrestes durch O oder S-Atome unterbrochen sein können, A -PO(OH)₂ (Phosphonsäuregruppe), -O-PO(OH)₂ (Dihydrogenphosphatgruppe), -O-PO(OH)R⁶ (Monohydrogenphosphatgruppe) oder -SO₃H (Sulfonsäuregruppe) ist, wobei R⁶ ein verzweigter oder vorzugsweise unverzweigter C₁-C₂₀-, vorzugsweise C₁-C₁₂-, insbesondere C₁-C₆-Alkylrest, ein C₃-C₁₀-, vorzugsweise C₆-C₁₀-Cycloalkylrest oder ein aromatischer C₆-C₂₀-, vorzugsweise C₆-C₁₀-Rest ist, wobei R⁶ substituiert oder unsubstituiert sein kann, w eine ganze Zahl von 1 bis 3 ist;
enthalten und das Polykondensat (1), das Monomer (3) oder beide durch Fluor substituiert sind, wobei das Material mindestens ein Polykondensat oder ein Monomer enthält, das durch Fluor substituiert ist.

Unter Halogen wird im Rahmen dieser Erfindung vorzugsweise -F, -Cl, -Br und -I verstanden. Alkylgruppen können verzweigt oder vorzugsweise unverzweigt sein. Unter Kohlenwasserstoffresten, die durch Heteroatome oder funktionelle Gruppen unterbrochen sein können, werden solche Reste verstanden, bei denen die Heteroatome oder Gruppen in die C-C-Kette integriert sind, d.h. beidseitig mit Kohlenstoffatomen verknüpft sind. Die Kohlenwasserstoffreste können aromatisch, aliphatisch oder aromatisch-aliphatisch sein. Bei Komponenten der oben definierten Art, die mehr als eine Gruppe mit der gleichen Bezeichnung enthalten, können diese Gruppen gleich oder verschieden sein. Für n = 3 kann das Silan (I) beispielsweise 3 gleiche Gruppen R¹ oder drei unterschiedliche Gruppen R¹ tragen.

Bei den optionalen Substituenten der Gruppe R² handelt es sich vorzugsweise um Estergruppen, insbesondere C₁-C₆-Alkylestergruppen, C₁-C₆-Alkyl- oder C₁-C₆-Alkoxyreste, Phenyl, -Cl, -Br und -OH, die optionalen Substituenten von R⁶ sind vorzugsweise C₁-C₆-Alkyl, C₁-C₆-Alkoxy, Halogen, vorzugsweise F, Cl oder Br, oder OH. Wenn R⁶ eine Alkylgruppe ist, ist diese vorzugsweise nicht durch weitere Alkylgruppen substituiert.

Die erfindungsgemäßen Werkstoffe sind Beschichtungsmaterialien, insbesondere für natürliche und künstliche Zähne, Zahnersatzteile und orthodontische Vorrichtungen, und werden daher im Folgenden auch als Dentalwerkstoffe bezeichnet. Die Anwendung der Werkstoffe kann intra- und extraoral erfolgen.

Bevorzugte radikalisch polymerisierbare Silane der Formel (I) sind wie folgt definiert:
- R¹ =: eine Vinyl-, (Meth)acryl-, Vinylcyclopropyl-, Allyl- oder Styryl-Gruppe,
- R² =: entfällt, Methylen, Ethylen, Propylen, Butylen, Phenylen, -(CH₂)₂CH-O-OC- (CH₂)₃-CO-NR⁹- (CH₂) ₃- oder - [CH₂-CHR⁹-O-OC-(CH2)₂₋]₂N-(CH₂)₃- mit R⁹ = H oder CH₃,
- R³ =: C₁-C₁₂-Alkyl, besonders bevorzugt C₁-C₃-Alkyl, insbesondere Methyl oder Ethyl;
- X =: F, Cl oder C₁-C₃-Alkoxy, besonders bevorzugt F, Methoxy oder Ethoxy;
- n =: 0 oder 1
- m =: 1.

Diese und die folgenden Angaben sind jeweils so zu verstehen, daß die bevorzugten und besonders bevorzugten Definitionen der einzelnen Variablen unabhängig voneinander gewählt werden können. Verbindungen bei denen alle Variablen eine der bevorzugten oder besonders bevorzugten Definitionen aufweisen, sind naturgemäß ganz besonders bevorzugt.

Beispiele für besonders bevorzugte Silane der Formel (I) sind 3-(Methacryloyloxy)-propyl-triethoxysilan, 3-(Methacryloyloxy)-propyl-trimethoxysilan, 3-(Acryloyloxy)propyl-methyldiethoxysilan, Vinyl- oder Allyl-trimethoxysilan, oder Umsetzungsprodukte von 3-Isocyanatopropyltriethoxysilan mit 2-Hydroxyethyl(meth)acrylat, Hydroxypropyl(meth)acrylat oder Glycerindimethacrylat.

Weiterhin bevorzugt sind Additionsprodukte von 3-Aminopropyltriethoxysilan oder 3-Mercaptopropyltriethoxysilan an Monomere die neben einer Acrylatgruppe eine oder mehrere Methacrylatgruppen enthalten, wie z.B. 2-Methacryloyloxyethylacrylat oder 2-Acryloyloxy-1,3-dimethacryloxypropan. Geeignet sind auch Amide die durch Umsetzung von 3-Aminopropyltriethoxysilan mit Methacrylatcarbonsäuren, z.B. Reaktionsprodukte von Bernsteinsäure- oder Glutarsäureanhydrid mit 2-Hydroxyethyl(meth)-acrylat, Hydroxypropyl(meth)acrylat oder Glycerindi(meth)-acrylat, zugänglich sind.

Bevorzugte Silane der Formel (II) sind wie folgt definiert:
- R⁴ =: lineares C₁-C₁₂-Alkyl, besonders bevorzugt C₁-C₆-Alkyl, insbesondere Methyl, Ethyl, Propyl, Phenyl, wobei die Wasserstoffatome dieser Reste ganz oder teilweise durch F ersetzt sind;
- Y =: F, Methoxy oder Ethoxy;
- p =: eine ganze Zahl von 1 bis 4, insbesondere 1 bis 3.

Besonders bevorzugte Beispiele für Silane der Formel (II) sind Tetrachloro-, Tetraethoxy- oder Tetramethoxysilan, CH₃-SiCl₃, CH₃-Si(OC₂H₅)₃, C₂H₅-SiCl₃, C₂H₅-Si(OC₂H₅)₃, Phenyl-Si (OC₂H₅)₃, C₃H₇-Si (OCH₃)₃, (CH₃)₂SiCl₂, (Phenyl) ₂SiCl₂, (CH₃)₂Si (OC₂H₅)₂, (C₂H₅)₃Si-Cl, (C₂H₅)₂Si (OC₂H₅)₂, (CH₃)₃Si-Cl, (CH₃O)₃Si-C₃H₆-NH₂, (CH₃O)₃Si-C₃H₆-SH, (CH₃O)₃Si-C₃H₆-NH₂. Silane mit p = 4 werden vorzugsweise in Kombination mit Silanen eingesetzt, bei denen p 1, 2 oder 3 ist.

Erfindungsgemäß sind solche Polykondensate (1) bevorzugt, die durch Fluor substituiert sind, vorzugsweise durch 3 bis 22 Fluoratome. Diese werden vorzugsweise dadurch erhalten, daß als Komponente (B) ein Silan der Formel (II) eingesetzt wird, das Fluorsubstituenten aufweist, wobei diese Fluorsubstituenten an den Rest R⁴ gebunden sind. Die Fluoratome sind vorzugsweise auf eine möglichst geringe Anzahl benachbarter Kohlenstoffatome konzentriert. Besonders bevorzugt sind Polykondensate, die Perfluoralkylreste des Typs C_{b}F_{2b+1}- oder -C_{b}F_{2b}- enthalten, wobei sich b aus der Definition der Silane der Formeln (I) und (II) ergibt.

Bevorzugte fluorsubstituierte Silane der Formel (II) sind CF₃CH₂CH₂-Si (OC₂H₅)₃, C₂F₅-CH₂CH₂-Si (OC₂H₅) ₃, C₄F₉CH₂CH₂-Si (OC₂H₅) ₃, n-C₆F₁₃CH₂CH₂-Si (OC₂H₅) ₃, n-C₈F₁₇-CH₂CH₂-Si (OC₂H₅)₃, CF₃CH₂CH₂-SiCH₃(OC₂H₅)₂ und n-C₆F₁₃CH₂CH₂-O-CO-NH-(CH₂)₃-Si(OC₂H₅)₃.

Bevorzugte Metallverbindungen der Formeln (III) und (IV) sind wie folgt definiert:
Z = C₁-C₄-Alkoxy oder Cl.

Besonders bevorzugte Beispiele für Verbindungen der Formel (III) sind Al(OCH₃)₃, Al(OC₂H₅)₃, Al(OC₃H₇)₃, Al(OC₄H₉) ₃ und AlCl₃.

Besonders bevorzugte Metallverbindungen der Formel (IV) sind ZrCl₄, Zr(OC₂H₅)₄, Zr(OC₃H₇)₄, Zr(OC₄H₉)₄, ZrOCl₂, TiCl₄, Ti(OC₂H₅)₄, Ti(OC₃H₇)₄ und Ti(OC₄H₉)₄.

Die Verbindungen der Formeln (III) und (IV) können durch Komplexbildung funktionalisiert werden, beispielsweise durch Komplexierung mit Säuren, vorzugsweise Propionsäure, Methacrylsäure, Maleinsäure oder Bernsteinsäure, oder β-Dicarbonylverbindungen, vorzugsweise Acetylaceton, Acetessigester oder 2-Acetoacetoxyethylmethacrylat. Bei der Komplexierung werden die Gruppen Z ganz oder teilweise durch die komplexierenden Gruppen der Komplexbildner verdrängt.

Zur Initiierung der radikalischen Photopolymerisation werden vorzugsweise Benzophenon, Acylphosphinoxide, wie 2,4,6-Trimethyl-benzoyldiphenylphosphinoxid, Benzoin sowie deren Derivate oder α-Diketone oder deren Derivate, wie 9,10-Phenanthrenchinon, 1-Phenyl-propan-1,2-dion, Diacetyl oder 4,4-Dichlorbenzil eingesetzt. Gemäß einer besonders bevorzugten Ausführungsform werden Campherchinon und 2,2-Methoxy-2-phenyl-acetophenon und α-Diketone in Kombination mit Aminen als Reduktionsmittel, wie z.B. 4-(Dimethylamino)-benzoesäureester (EMBO), N,N-Dimethylaminoethylmethacrylat, N,N-Dimethyl-sym.-xylidin oder Triethanolamin, verwendet.

Als thermische Initiatoren eignen sich besonders Azoverbindungen, wie Azobis(isobutyronitril) (AIBN) oder Azobis-(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid. Als Initiatoren für die Heißhärtung eignen sich auch Benzpinakol und 2,2'-Dialkylbenzpinakole.

Als Initiatoren für die Polymerisation bei Raumtemperatur werden vorzugsweise Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Kombinationen von Peroxiden mit solchen Reduktionsmitteln, wie z.B. Ascorbinsäure, Barbiturate oder Sulfinsäuren, geeignet.

Als Komponente (3) können die erfindungsgemäßen Werkstoffe organische, radikalisch polymerisierbare Monomere enthalten, insbesondere ein oder mehrere (Meth)acrylate, (Meth)acrylamide und/oder Pyrrolidonderivate. Hierzu eignen sich insbesondere bei Raumtemperatur flüssige Monomere (Verdünnermonomere), die nur eine polymerisierbare Gruppe aufweisen, und Monomere, die zwei oder mehr, vorzugsweise 2 bis 5 polymerisierbare Gruppen aufweisen und eine Vernetzung des Polymerisats bewirken (Vernetzermonomere). Monomere mit zwei oder mehr polymerisierbaren Gruppen werden auch als mehrfunktionelle Monomere bezeichnet. Die Komponente (3) enthält vorzugsweise mindestens ein Vernetzermonomer. Sowohl Verdünnermonomere als auch Vernetzermonomere können durch ein oder mehrere, vorzugsweise 3 bis 22 Fluoratome substituiert sein. Die Verwendung fluorierter, d.h. fluorsubstituierter Monomere ist bevorzugt, bei der Verwendung nicht fluorierter Polykondensate (1) zwingend.

Die Fluoratome sind auch hier vorzugsweise auf eine möglichst geringe Anzahl benachbarter Kohlenstoffatome konzentriert. Besonders bevorzugt sind Monomere, die Perfluoralkylreste des Typs C_{b}F_{2b+1}- oder -C_{b}F_{2b}- enthalten, wobei sie b aus der Definition der Monomere ergibt.

Besonders bevorzugte Verdünnermonomere sind Mono(meth)-acrylate, z.B. Methyl-, Ethyl-, Butyl-, Benzyl-, Furfuryl- oder Phenyl(meth)acrylat und Mesitylmethacrylat, das sich durch eine hohe Hydrolysestabilität auszeichnet, und 2-Hydroxyethyl-methacrylat (HEMA). Weitere bevorzugte Beispiele sind N-monosubstituierte oder N-disubstitiuierte Acrylamide, wie N-Ethylacrylamid, N,N-Dimethacrylamid, N-(2-Hydroxyethyl)acrylamid oder N-Methyl-N-(2-hydroxyethyl)-acrylamid, N-monosubstituierte Methacrylamide, wie N-Ethylmethacrylamid oder N-(2-Hydroxyethyl)methacrylamid, und N-Vinylpyrrolidon, wobei diese Verbindungen ebenfalls eine hohe Hydrolysebeständigkeit aufweisen.

Bevorzugte Vernetzermonomere sind mehrfunktionelle Acrylate und Methacrylate, wie Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglycoldi(meth)acrylat, Decandioldi(meth)acrylat, Trimethylolpropantri(meth)acrylat, Pentaerythrittetra(meth)acrylat, Butandioldi(meth)acrylat, 1,10-Decandioldi(meth)acrylat, 1,12-Dodecandioldi(meth)-acrylat, vernetzende Pyrrolidone, wie 1,6-Bis(3-vinyl-2-pyrrolidonyl)-hexan, und Bisacrylamide, wie Methylen- oder Ethylenbisacrylamid, Bismethacrylamide, wie N, N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können oder kommerziell erhältlich sind.

Bevorzugte fluorierte monofunktionelle Monomere sind 2,2,2-Trifluorethyl(meth)acrylat, Pentafluorethylmethacrylat, 2-(Pentafluorbutyl)ethyl(meth)acrylat, 2,2,3,3-Tetrafluorpropyl-(meth)acrylat, 3-(Pentafluorbutyl)-2-hydroxypropyl(meth)-acrylat, Perfluorcyclohexylmethylmethacrylat, 3-(Perfluorhexyl)-2-hydroxypropyl(meth)acrylat, 2-(Perfluor-3-methylbutyl)ethylmethacrylat, 3-(Perfluor-3-methylbutyl)-2-hydroxypropyl(meth)acrylat, 1H,1H,5H-Octafluorpentyl(meth)acrylat, 1H,1H,2H,2H-Pentafluordecylacrylat, 1H,1H-Perfluor-n-decyl-(meth)acrylat, 2-(Perfluordecyl)ethyl(meth)acrylat, 2-(Perfluor-9-methyldecyl)ethyl(meth)acrylat, 2-Perfluor-5-methylhexyl)ethyl(meth)acrylat, 2-(Perfluor-7-methyloctyl)ethyl(meth)-acrylat, 1H,1H,7H-Dodecafluorheptyl(meth)acrylat, 1H,1H-Perfluoroctyl(meth)acrylat, 1H,1H,2H,2H-Perfluoroctyl(meth)acrylat, 1H,1H,9H-Hexadecafluornonyl(meth)acrylat und 1H,1H,11H-Eicosafluorundecyl(meth)acrylat und 1H,1H,2H,2H-Pentafluordecylacrylat.

Bevorzugte fluorierte Vernetzermonomere sind fluoriertes Triethylenglycoldimethacrylat (TEGDMA-F), 2,2,3,3-Tetrafluor-1,4-butandioldimethacrylat, 1H,1H,6H,6H-Perfluor-1,6-hexandioldi-(meth)acrylat, 1H,1H,10H,10H-Perfluordecandioldi(meth)acrylat, 2,2,3,3,4,4,5,5,6,6,7,7-Dodecafluor-1,8-octandioldi(meth)-acrylat und fluoriertes Bis-GMA (Bis-GMA-F: 2,2-Bis[(4-(2-hydroxy-3-methacryloyloxy)propyloxy)phenyl]-hexafluorpropan):

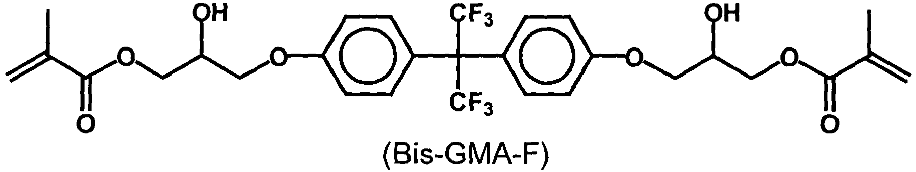

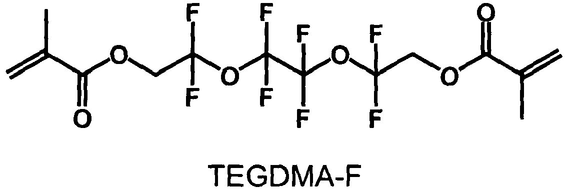

Unter organischen, radikalisch polymerisierbaren Monomeren (3) werden demnach solche Verbindungen verstanden, die keine Silane sind und keine der für Verbindungen der Formel (V) definierten Säuregruppen tragen.

Bevorzugte acide Monomere (4) der Formel (V) sind wie folgt definiert:

R⁵ₜ-Sp-A_{w} (V)

- R⁵ =: eine Vinyl-, (Meth)acryl-, (Meth)arylamid-, Styryl-Gruppe oder eine Gruppe der Formel (VI) 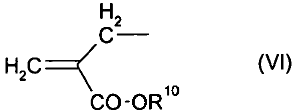 in der R¹⁰ H oder ein verzweigter oder vorzugsweise unverzweigter C₁-C₂₀-, vorzugsweise C₁-C₁₂-, insbesondere C₁-C₆-Alkylrest, ein C₃-C₂₀-, vorzugsweise C₃-C₆-Cycloalkylrest, oder ein aromatischer C₆-C₂₀-, vorzugsweise C₆-C₁₀-Rest ist, wobei R¹⁰ substituiert oder unsubstituiert sein kann;
- t =: 1 oder 2;
- Sp =: C₁-C₁₅-Kohlenwasserstoffrest, der durch O-Atome, vorzugsweise 1 bis 5 O-Atome, unterbrochen sein kann;
- A =: -O-P(O)(OH)₂, -SO₃H, insbesondere -PO(OH)₂;
- W =: 1 oder 2.

Bei den optionalen Substituenten des Restes R¹⁰ handelt es sich vorzugsweise um C₁-C₆-Alkyl, C₁-C₃-Alkoxy, Halogen oder OH. Wenn R¹⁰ ein Alkylrest ist, ist dieser vorzugsweise nicht durch weitere Alkylreste substituiert.

Monomere der Formel (V) werden im Folgenden auch als acide Monomere bezeichnet.

Zu den bevorzugten polymerisationsfähigen Mono- und Dihydrogenphosphorsäureestern gehören 2-Methacryloyloxyethyl-phenyl-hydrogenphosphat, Dipentaerythritol-pentamethacryloyloxyphosphat, 10-Methacryloyloxydecyl-dihydrogenphosphat, Dipentaerythritol-pentamethacryloyloxyphosphat und 6-(Methacrylamido)hexyldihydrogenphosphat.

Eine bevorzugte Phosphonsäure ist 2-[2-Dihydroxyphosphoryl)-ethoxymethyl]-acrylsäure-2,4,6-trimethyl-phenylester.

Bevorzugte polymerisationsfähige Sulfonsäuren sind Vinylsulfonsäure, 4-Vinylphenylsulfonsäure und 3-(Methacrylamido)-propylsulfonsäure.

Ganz besonders bevorzugte Monomere der Formel (V) sind die Phosphonsäuren Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure.

Polymerisierbare Carbonsäuren, d.h. Monomere, die als acide Gruppe allein eine Carboxylgruppe enthalten, sind als acide Monomere nicht geeignet. Gemäß einer bevorzugten Ausführungsform enthalten die erfindungsgemäßen Dentalmaterialien keine polymerisierbaren Carbonsäuren, wie (Meth)acrylsäure, und vorzugsweise auch keine nicht polymerisierbaren Carbonsäuren.

Die erfindungsgemäßen Werkstoffe können neben den bereits genannten Komponenten vorteilhaft weitere Komponenten, insbesondere Füllstoff (5), enthalten. Durch die Zugabe von Füllstoff lassen sich die mechanischen Eigenschaften der Beschichtung verbessern. Bevorzugt sind Füllstoffe mit einer mittleren Partikelgröße von 5 bis 60 nm, vorzugsweise 10 bis 40 nm. Wenn nicht anders angegeben handelt es sich bei der Partikelgröße jeweils um die durch dynamische Lichtstreuung ermittelte Partikelgröße. Besonders bevorzugte Füllstoffe sind partikuläres SiO₂, Al₂O₃, Ta₂O₅, Yb₂O₃, ZrO₂, nanopartikuläres Silber, TiO₂ und Mischoxide aus SiO₂, ZrO₂ und/oder TiO₂. Besonders geeignet sind nanopartikuläre Füllstoffe mit einer mittleren Partikelgröße kleiner 100 nm, die mit polymerisationsfähigen Gruppen oberflächenmodifiziert sind und auf Grund ihrer geringen Partikelgröße nicht die Transparenz der Beschichtung verringern, d.h. nicht agglomerieren. Eine Oberflächenmodifizierung wird bei silikatischen Füllstoffen vorzugsweise durch Silanisieren erreichen, bei nicht silikatischen Füllstoffen auf die in der WO 00/69392 beschriebene Weise.

Die Füllstoffmenge beträgt vorzugsweise 0 bis 50 Gew.-%, besonders bevorzugt 0 bis 20 Gew.-% und insbesondere 0 bis 10 Gew.-%. Alle Angaben beziehen sich auf die Gesamtmasse des Dentalwerkstoffs.

Darüber hinaus können die erfindungsgemäßen Werkstoffe weitere Additive enthalten, wie z.B. Farbmittel (Pigmente oder Farbstoffe), Stabilisatoren, Aromastoffe, mikrobiocide Wirkstoffe, Lösungsmittel, Weichmacher oder UV-Absorber. Bevorzugte Lösungsmittel sind Wasser, Ethanol, Aceton und Ethylacetat. Durch die Zugabe flüchtiger Lösungsmittel, wie Aceton oder Ethanol, können das Benetzungsverhalten und die Filmbildung positiv beeinflußt werden. Diese Lösungsmittel können allein oder in Mischung mit Wasser eingesetzt werden.

Zemente und Füllungskomposite enthalten in der Regel kein Lösungsmittel, Adhäsive und Beschichtungsmaterialien vorzugsweise 0 bis 20 Gew.-%, besonders bevorzugt 0 bis 10 Gew.-% und ganz besonders bevorzugt 0 bis 5 Gew.-% Lösungsmittel, jeweils bezogen auf die Gesamtmasse des Dentalwerkstoffs.

Erfindungsgemäß besonders bevorzugt sind Werkstoffe, die vollständig aus den hierin definierten Komponenten und insbesondere den hierin definierten bevorzugten Komponenten zusammengesetzt sind.

Die Polykondensate (1) lassen sich durch hydrolytische Polykondensation der Komponenten A bis C auf die nachfolgend beschriebene Weise erhalten, wobei die Komponente (A) allein oder zusammen mit den Komponenten (B) und/oder (C) zur Herstellung der Polykondensate verwendet werden kann. Gemäß einer bevorzugten Ausführungsform werden zur Herstellung der Polykondensate Mischungen eingesetzt, die neben der Komponente (A) mindestens eine der Komponenten (B) oder (C), vorzugsweise (B) und (C) enthalten. Besonders bevorzugte Ausgangsmischungen zur Herstellung der Polykondensate enthalten 30 bis 70 Gew.-% (A) und 5 bis 70 Gew.-% (B), insbesondere 40 bis 65 Gew.-% (A) und 15 bis 65 Gew.-% (B), ganz besonders bevorzugt 45 bis 60 Gew.-% (A) und 20 bis 60 Gew.-% (B). Die Komponente (C) wird, wenn vorhanden, vorzugsweise in einer Menge von 5 bis 30 Gew.-% , besonders bevorzugt 10 bis 20 Gew.-% (C) eingesetzt. Die Mengenangaben beziehen sich auf die Gesamtmasse der zur Herstellung der Polykondensate (1) verwendeten Komponenten. Werden als Edukte nur Silane eingesetzt, erfolgt die hydrolytische Kondensation vorzugsweise so, daß man die zu hydrolysierenden Verbindungen, allein oder in Gegenwart von Lösungsmittel und gegebenenfalls eines Hydrolyse- und Kondensationskatalysators, mit einer stöchiometrischen Menge an Wasser oder einem Wasserüberschuß versetzt. Die Umsetzung wird bei Raumtemperatur, unter schwachem Kühlen oder gelindem Erwärmen durchgeführt. Die Reaktionsmischung wird solange gerührt bis die Hydrolyse und Kondensation zum gewünschten Umsatz abgelaufen sind, d.h. die monomeren Ausgangsverbindungen in der Reaktionsmischung praktisch vollständig aufgebraucht, d.h. durch ²⁹Si-NMR-Spektroskopie nicht mehr nachweisbar sind. Der Verlauf der hydrolytischen Kondensation läßt sich mittels ²⁹Si-NMP-Spektoskopie verfolgen.

Werden neben den Silanen Metallverbindungen (C), insbesondere Verbindungen des Zr oder Al, verwendet, wird das Wasser vorzugsweise in Portionen bei Temperaturen von -30 °C bis Raumtemperatur zugegeben. Als Lösungsmittel eignen sich besonders niedere aliphatische Alkohole, insbesondere Ethanol, oder Isopropanol, aliphatische Ketone, insbesondere Aceton, Ester, insbesondere Essigsäureethylester, Ether, insbesondere Diethylether oder Tetrahydrofuran, DMF und Amine. Als katalytische Säuren können vorteilhaft Mineralsäuren, insbesondere Salzsäure oder Flußsäure, Carbonsäuren, insbesondere Ameisensäure oder Essigsäure, Sulfonsäuren, Phosphonsäuren und Phosphorsäure verwendet werden. Vorzugsweise wird die Kondensation jedoch allein durch die aciden, radikalisch polymerisierbaren Monomere (V) initiiert.

Im Rahmen der Erfindung wurde überraschenderweise gefunden, daß vor allen polymerisationsfähige Dihydrogenphosphatester und insbesondere Phosphonsäuren nicht nur eine sehr schnelle hydrolytische Kondensation z.B. der Silane bewirken sondern gleichzeitig eine Benetzung der Zahnhartsubstanz fördern und die Haftung auf der Zahnhartsubstanz verbessern.

Gemäß einer bevorzugten Ausführungsform erfolgt die Herstellung der Polykondensate (1) durch Acidolyse von Metallalkoxiden, z.B. durch Kondensation von Trialkoxysilanen, Tetraalkoxysilanen, Titanaten und Zirkonaten unter Zugabe von Carbonsäuren, wie z.B. Ameisensäure (vgl. z.B. WO 93/2333 oder DE 101 06 787 A1). Bei dieser Reaktion entsteht das zur hydrolytischen Kondensation erforderliche Wasser durch Umsetzung der Alkoxide mit der Säure unter gleichzeitiger Bildung von Metalloxid und Ester.

Die Herstellung der Polykondensate kann alternativ auch in Anwesenheit der übrigen Komponenten hergestellt werden. Hierzu werden die Komponenten (A) und ggf. (B) und/oder (C) mit radikalisch polymerisierbarem Monomer (4) und ggf. (3), Lösungsmittel und Füller gemischt und dann durch Zugabe von Säure und Wasser die Kondensationsreaktion eingeleitet. Anschließend wird das polykondensathaltige Gemisch mit einem Initiator für die radikalische Polymerisation versetzt und durch radikalische Polymerisation gehärtet.

In Abhängigkeit von der Art und Anzahl der hydrolysierbaren Gruppen der Silane bilden sich Polykondensate, die flexible- oder glasartige Eigenschaften zeigen. Die Vernetzungsdichte und damit die Härte können beispielsweise durch die Verwendung von Tetraalkoxysilanen wie Tetraethoxysilan erhöht werden. Erhöht man den Anteil an organischen Substituenten, beispielsweise durch organisch modifizierte Trialkoxysilane oder Dialkoxysilane, wird das Netzwerk zunehmend flexibler, wobei auch Art und Größe der organischen Reste eine Rolle spielen. Durch den Einbau von Zr-, Ti- oder Al-Verbindungen können die Härte, Abrasivität und der Brechungsindex der Beschichtung gesteuert werden. Durch Cokondensation von Silanen mit Metallalkoxiden werden Materialien mit keramischen Eigenschaften wie z.B. hoher Härte erhalten. Aluminiumoxid führt zu einer Verringerung der Abrasivität, der Einbau der Elemente Titan und Zirkonium bewirkt eine Erhöhung des Brechungsindexes, so daß dieser durch die Menge an entsprechenden Ti- und/oder Zr-Verbindungen gezielt eingestellt werden kann. Der Brechungsindex ist darüber hinaus von der Porosität und Kristallinität der gebildeten Polykondensate abhängig.

Generell wird die Polykondensation vorzugsweise so gesteuert, daß bei Raumtemperatur flüssige Polykondensate erhalten werden, da diese bevorzugt sind. Die Homopolymerisation von Trialkoxysilanen ergibt meist fließfähige Produkte, feste Produkte lassen sich beispielsweise durch Zugabe von Tetraalkoxysilanen erhalten.

Nach Abschluß der Kondensationsreaktion werden die Polykondensate mit den übrigen Komponenten der erfindungsgemäßen Werkstoffe gemischt. Unter Polykondensaten werden Kondensate mit mehr als 2, vorzugsweise mehr als 5 und besonders bevorzugt mehr als 10 Silicium-, Aluminium-, Zirkonium- und/oder Titanatomen verstanden. Die Werkstoffe enthalten vorzugsweise keine monomeren Silane oder Disiloxane.

Die erfindungsgemäßen Werkstoffe können in Abhängigkeit des gewählten Polymerisationsinitiators beispielsweise thermisch oder photochemisch gehärtet werden. Die Eigenschaften der gehärteten Materialien lassen sich durch verschiedene Faktoren variieren. Die mechanischen Eigenschaften, wie Festigkeit und Flexibilität lassen sich zum einen durch die Netzwerkdichte der gebildeten Kondensate (hoher Anteil an hydrolysierbaren Gruppen der Verbindungen (A) bis (C) führt zu einer hohen Netzwerkdichte) und Polymerisate (Monomere mit mehreren polymerisierbaren Gruppen führen zu einem dichten dreidimensionalen Netzwerk) steuern.

Aber auch die organischen Spacer können das Netzwerk beispielsweise durch Ausbildung von Wasserstoffbrückenbindungen, Variation ihrer Länge und ihrer funktionellen Gruppen, die mehr oder weniger flexibel sein können, steifer oder flexibler machen. Andere Eigenschaften, wie die Brechzahl, lassen sich durch die Spacergrupppen oder z.B. auch durch den Einbau von Metallatomen, wie Zr und Ti, variieren. Bei aliphatischen Spacergruppen nimmt mit zunehmender Länge der Spacergruppe die Flexibilität der Materialien zu, der Einbau aromatischer Spacergruppen vergrößert dagegen die Steifigkeit der gehärteten Materialien. Durch Spacer mit hydrophilen oder hydrophoben Gruppen läßt sich der Wasseraufnahmefähigkeit der Werkstoffe erhöhen oder reduzieren.

Durch die Variation der Vernetzungsdichte, d.h. durch Veränderung der Anzahl der polymerisationsfähigen Gruppen der Silane oder der gegebenenfalls weiter zugesetzten organischen Monomere ist eine weitere Beeinflussung der Eigenschaften der Beschichtung möglich.

Die erfindungsgemäßen Werkstoffe enthalten mindestens ein Polykondensat oder ein radikalisch polymerisierbare Monomer, das durch Fluor substituiert ist. Gemäß einer bevorzugten Ausführungsform enthalten die Werkstoffe sowohl ein Polykondensat als auch ein radikalisch polymerisierbares Monomer, das durch Fluor substituiert ist. Durch die Verwendung von fluorhaltigen Komponenten, insbesondere von Fluorsilanen und fluorierten Monomeren, kann eine Verringerung der Oberflächenenergie der Materialien und damit eine Verminderung der Anlagerung von Plaque realisiert werden. Da sich diese Komponenten in der Regel an der Grenzschicht zur Luft anreichern, ist nur ein vergleichsweise geringer Anteil an diesen Komponenten erforderlich, um die gewünschte Wirkung zu erzielen. Werkstoffe, die bezogen auf das Gesamtgewicht der gehärteten Zusammensetzung 5 bis 10 Gew.-% Fluor enthalten, sind bevorzugt.

Auf der anderen Seite verleihen die fluorhaltigen Komponenten den Werkstoffen hydrophobe Eigenschaften, was eine Benetzung der Zahnoberfläche deutlich erschwert und die Haftung der Materialien an der Zahnoberfläche beeinträchtigt. Die erfindungsgemäßen Werkstoffe weisen diese Nachteile nicht auf. Durch die Abmischung der oben genannten Komponenten ist es gelungen, Werkstoffe herzustellen, die einerseits wirkungsvoll die Anlagerung beispielsweise von Plaque verhindern, andererseits aber ein gutes Benetzungsvermögen für hydrophile Oberflächen und gute Haftungseigenschaften zeigen, d.h. zwei an sich entgegengesetzte Eigenschaften miteinander vereinen. Dies ist eine überraschende Verbesserung gegenüber bekannten Materialien.

Die erfindungsgemäßen Werkstoffe werden beispielsweise durch Aufsprühen, Tauchen oder Streichen, z.B. mit einem Pinsel, auf die zu beschichtende Oberfläche aufgebracht. Der Auftrag kann ein- oder mehrfach erfolgen. Bei mehrfachem Auftrag ist es vorteilhaft, die vorher aufgetragene Schicht zunächst zu trokkenen, beispielsweise durch Abdampfen des ggf. vorhandenen Lösungsmittels und/oder durch radikalische Polymerisation, und erst dann die nächste Schicht aufzubringen. Nach dem Auftrag werden die Materialien durch radikalische Polymerisation gehärtet.

Die Werkstoffe eignen sich besonders zur Beschichtung von künstlichen und insbesondere natürlichen Zähnen und Zahnersatzteilen. Künstliche Zähne enthalten in der Regel Kompositmaterialien mit einer (Meth)acrylat-Polymermatrix und Füllstoff, meist auf Basis von SiO₂. Darüber hinaus sind die Werkstoffe auch zur Beschichtung von dentalen Kunststoffen, wie Prothesenkunststoffen auf Basis von Polymethylmethacrylat (PMMA), Materialien für temporäre Kronen und Brücken auf PMMA- oder Kompositbasis, Kompositfüllungsmaterialien, Kompositverblend- und Gerüstmaterialien, kieferorthopädischen Platten und Aktivatoren (PMMA-Basis), dentalen Metalllegierungen und Keramiken geeignet. Soweit es sich nicht um natürliche Zähne handelt, haben die Substrate vorzugsweise die Form von dentalen Prothesen, wie Voll- oder Teilprothesen, künstlichen Zähnen oder orthodontischen Vorrichtungen. Verfahren zur Beschichtung solcher Substrate sind ebenfalls Gegenstand der Erfindung. Diese umfassen die Schritte:
(i) vorzugsweise Reinigen der zu behandelnden Oberfläche, beispielsweise durch Entfetten oder Polieren;
(ii) Aufbringen eines erfindungsgemäßen Werkstoffs;
(iii) Härten des aufgebrachten Werkstoffs durch radikalische Polymerisation;
(iv) vorzugsweise Entfernen der Inhibierungsschicht.

Das erfindungsgemäße Verfahren zeichnet sich dadurch aus, daß abgesehen von der optionalen Reinigung keine Vorbehandlung der Oberfläche durch Grundieren mit einem Primer erforderlich ist. Besonders vorteilhaft ist, daß bei der Behandlung natürlicher Zähne keine Konditionierung der Zahnoberfläche durch Säurebehandlung und folglich kein Schutz nicht zu behandelnder Zahnoberflächen mit Schutzüberzügen erforderlich ist.

Unter einer Inhibierungsschicht wird eine dünne Schicht ungehärteten Materials verstanden, die dadurch entsteht, daß Luftsauerstoff in die Oberfläche des Materials diffundiert, in der Regel bis zu einer Tiefe von etwas 100 µm, und dort die Polymerisation inhibiert. Diese Inhibierungsschicht wird vorzugsweise mit einem geeignetem Lösungsmittel wie einem Alkohol, vorzugsweise einer wäßrigen alkoholischen Lösung, besonders bevorzugt einer ethanolischen Lösung entfernt. Die Bildung einer Inhibierungsschicht kann durch geeignete Maßnahmen, die den Zutritt von Sauerstoff zur behandelten Oberfläche unterbinden, verhindert werden, beispielsweise durch das Auflegen einer sauerstoffundurchlässigen Folie oder durch das Arbeiten in einer sauerstofffreien Atmosphäre.

Die Behandlung natürlicher Zähne erfolgt vorzugsweise auf die im Folgenden beschriebene Weise. Die Behandlung kann zur therapeutischen Zwecken, beispielsweise zur prophylaktischen Behandlung von Karies oder Parodontitis, Gingivitis, Hypersensibilitäten, oder zu rein kosmetischen Zwecken, z.B. zur Verhinderung von Zahnverfärbungen oder zur Aufhellung von verfärbten Zähnen, dienen. Weiter kann die Behandlung dem Schutz der Zähne bei Zahnbehandlungen oder dem mechanischen Schutz vor Alterungsprozessen, wie Abrasion und Rißbildungen, dienen. Für Behandlungen, die intraoral durchgeführt werden, sind photochemisch härtbare Werkstoffe bevorzugt.

Vor der Applikation der erfindungsgemäßen Dentalwerkstoffe, vor allem auf Karies gefährdete Bereiche der Zahnoberfläche, wie den Approximalflächen, Bereiche nahe der Gingiva und freiliegende Zahnhälse, wird die Zahnoberfläche gründlich gereinigt, z.B. mit einem Gummikelch und einer Reinigungspaste, wie sie für die professionelle Zahnreinigung eingesetzt werden. Vorhandener Zahnstein wird gegebenenfalls beseitigt. Anschließend werden Reste der Reinigungspaste entfernt, die zu behandelnden Zahnoberflächen mit Wasser gründlich abgespült und dann mit einem Luftstrahl getrocknet. Vorzugsweise wird durch das Einbringen von Watterollen eine Kontamination der Oberflächen mit Speichel verhindert. Danach wird der Dentalwerkstoff in Form einer dünnen Schicht auf die Oberflächen aufgebracht, z.B. mit einem so genannten Microbrush, einem weichen Pinsel oder anderen geeigneten Hilfsmitteln. Nach einer Einwirkzeit von 10 - 20 Sekunden wird jede beschichtete Zahnfläche 20 - 40 Sekunden (abhängig von der Lampenleistung) mit einer im Dentalbereich gängigen Polymerisationslampe bestrahlt. Anschließend werden ggf. weitere Schichten des Werkstoffs aufgebracht und dann, nach dem entfernen der Watterollen, nochmals mit Wasser gespült.

Weiterer Gegenstand der Erfindung sind Kits, die alle zur Anwendung der erfindungsgemäßen Werkstoffe notwendigen Materialien enthalten. Bevorzugt sind Kits, die neben dem polymerisierbaren Werkstoff eine Applikationshilfe, z.B. einen Pinsel oder einen Brush enthalten. Der oder die Werkstoffe selbst sind dabei in geeigneten, d.h. vorzugsweise luft- und lichtdichten Behältern untergebracht.

Die Erfindung wird im folgenden anhand von Ausführungsbeispielen näher erläutert.

### Beispiel 1: Herstellung von Dentalwerkstoffen auf der Basis von fluorierten Silanen und aciden Monomeren

Zur Herstellung der in Tabelle 1 gezeigten Werkstoffe wurden jeweils drei Lösungen angesetzt und diese anschließend miteinander gemischt.

Lösung 1 bestand aus den jeweils aufgeführten Mengen an MEMO, PFOTEOS und 40 % der angegebenen Menge an HEMA bzw. MMA. Bei den Proben 2 und 4 wurde zusätzlich monodisperses SiO₂ (13 nm Primärpartikelgröße) dispergiert in TEGDMA als Füllstoff zugegeben.
Lösung 2 bestand aus den jeweils aufgeführten Mengen an BisGMA und 40% der angegebenen Menge an HEMA bzw. MMA.

Lösung 3 bestand aus den jeweils aufgeführten Mengen an MA-154 und 20% der Menge an HEMA bzw. MMA.

Lösung 1 wurde mit Lösung 2 vermischt, dann Lösung 3 zugegeben und die gesamte Mischung 5 min bei Raumtemperatur und weitere 2 min bei 50°C gerührt. Hierbei findet eine Kondensation der Silane MEMO und PFOTEOS statt. Die Kondensation wurde ohne Wasserzusatz durchgeführt, und es wurden relativ dünnflüssige Lacke erhalten. Die Proben wurden zusätzlich mit 0.5 Gew.% 4-(Dimethylamino)-benzoesäureester, 0,4 Gew.% Campherchinon und 0,2 Gew.% 2,4,6-Trimethyl-benzoyldiphenylphosphinoxid (Lucerin TPO) versetzt, so daß die Werkstoffe mit Blaulicht gehärtet werden konnten.

**Tabelle 1**

| **Dentalwerkstoffe auf der Basis monomerer Silane und acider Monomere** | | | | | | | | | |
|---|---|---|---|---|---|---|---|---|---|
| **Nr.** | **Zusammensetzung (Angaben in Gew.-%)** | | | | | | | | |
| | BisGMA | MMA | HEMA | TEGDMA | MEMO | PFOTEOS | MA154 | Füller | Initiator |
| 1 | 21.27 | 21.27 | - | - | 20.88 | 21.24 | 14.24 | - | 1,1 |
| 2 | 15,96 | 16.18 | - | 15.37 | 15.81 | 16.08 | 11.21 | 8.29 | 1,1 |
| 3 | 22.64 | - | 20,79 | - | 20.70 | 20.96 | 13.81 | - | 1,1 |
| 4 | 16.47 | - | 16,85 | 12.48 | 15.91 | 16.12 | 12.75 | 8.32 | 1,1 |

| | | | | | | | | | |
|---|---|---|---|---|---|---|---|---|---|
| BisGMA: 2,2-Bis-[4-(2-hydroxy-3-methacryloxy-propoxy)phenyl)propan MMA: Methylmethacrylat HEMA: 2-Hydroxyethyl-methacrylat TEGDMA: Triethylenglykoldimethacrylat MEMO: Methacryloxypropyltrimethoxysilan PFOTEOS: Tridecafluoroctyltriethoxysilan MA154: 2-[4-(Dihydroxyphosphoryl)-2-oxa-butyl]-acrylsäure | | | | | | | | | |

### Beispiel 2: Herstellung eines Dentalwerkstoffs auf der Basis von fluoriertem Silan (Vergleichsbeispiel)

Es wurde ein Dentalmaterial der folgenden Zusammensetzung hergestellt. Dieses enthielt als fluoriertes Silan Tridecafluoroctyltriethoxysilan (PFOTEOS) jedoch kein acides Monomer im Sinne der Erfindung. Statt dessen wurde ein nicht erfindungsgemäßes acides Monomer (Methacrylsäure) eingesetzt. Die Herstellung des Materials erfolgte in Analogie zu DE 41 18 184 A1 (Beispiel 1).

**Tabelle 2**

| **Dentalwerkstoff auf der Basis von monomerem Silan (Vergleichsmaterial)** | |
|---|---|
| **Komponente** | **Anteil [mol]** |
| MEMO | 0,9 |
| PFOTEOS | 0,1¹ |
| Methanol | 3,0 |
| Wasser | 1,6 |
| Zr(OPr)₄² | 0,1³ |
| Methacrylsäure | 0,2 |

| | |
|---|---|
| ¹ entspricht 10 Gew.-% fluorhaltiges Silan ² 70 %ig in PrOH ³ bezogen auf Zr | |

### Beispiel 3: Bestimmung des Abrasionsverhaltens

Für die Untersuchung des Abrasionsverhaltens wurden gesäuberte, extrahierte Rinder-Schneidezähne verwendet. Die Zahnoberfläche wurde mit Wasser gespült und im Luftstrom getrocknet. Auf die Zähne wurden die Lacke Nr. 1 bis Nr. 4 aufgetragen und zur Härtung für 20 s mit einer Polymerisationslampe (Astralis 7) bestrahlt. Anschließende wurden die so präparierten Schneidezähne in einem Zahnbürstensimulationsgerät (ZBS) einer vierstündigen kreisförmigen Zahnbürstenbewegung ausgesetzt (Auflagekraft 1 N). Hierbei wurde ein Zahnpastenbrei verwendet, der in seiner Scheuerwirkung einer handelsüblichen Zahncreme entsprach (RDA 75). Der Verschleiß nach der simulierten Zahnbürstenbehandlung wurde in Analogie zu der in DIN ISO 11609 beschriebenen Methode ermittelt. Diese Behandlung entspricht einer Zahnreinigung über einen Zeitraum von etwa 2 Jahren, wenn man annimmt, daß jeder Zahn zweimal täglich für je 10 sec gereinigt wird. Bei allen untersuchten Proben war die auf den Zahn aufgebrachte Lackschicht nach 4 Stunden weitgehend abgetragen. Bei keiner der untersuchten Proben kam es jedoch zu einer großflächigen Ablösung des Lackes von der Zahnoberfläche. Die Adhäsion der Zahnlacke auf der Zahnoberfläche war während des gesamten Abrasionstests gegeben.

### Beispiel 4: Bestimmung der Haftung an Rinderzahnschmelz

Zur Untersuchung der Haftung der Werkstoffe an Dentin wurden Rinderzähne mit dem Werkstoff Nr. 1 behandelt und die Ergebnisse mit der Schmelzhaftung von kommerziell erhältlichen, lichthärtenden, einkomponentigen Zahnlacken auf der Basis von multifunktionellen Acrylaten und Methacrylaten (Luxatemp® Glaze & Bond, DMG, Hamburg; Heliobond®, Ivoclar Vivadent AG, Liechtenstein) verglichen. Darüber hinaus wurde ein Polysiloxan gemäß DE 41 33 494 hergestellt und getestet. Die Rinderzähne wurden so in Kunststoffzylinder eingebettet, daß sich das Dentin und der Kunststoff in einer Ebene befanden. Dann wurden die Zahnoberflächen gründlich mit Wasser abgespült. Anschließend wurde auf die Zähne mit einem Microbrush eine Schicht des Werkstoffs Nr. 1 bzw. der handelsüblichen Zahnlacke aufgetragen, die Lackschicht mit einem Luftstrahl kurz getrocknet und für 40 s mit einer Halogenlampe (Astralis 7, Vivadent) bestrahlt. Die Zahnoberflächen wurden nicht mit Säure konditioniert. Auf die Lackschicht wurde ein Kompositzylinder aus Tetric® Ceram (Ivoclar Vivadent) in zwei Schichten von je 1-2 mm polymerisiert. Die so behandelten Zähne wurden für 24 h bei 37 °C in Wasser gelagert und in Anschluß daran die Scherhaftfestigkeit gemäß ISO/TS11405 bestimmt. Die gefundenen Meßwerte sind in Tabelle 3 gezeigt.

**Tabelle 3**

| **Haftung an Rinderzahnschmelz (ohne Säurebehandlung)** | |
|---|---|
| **Werkstoff** | **Haftwert [MPa]¹** |
| Werkstoff Nr. 1 | 5.8 ± 1,0 |
| Luxatemp® Glaze & Bond² | 0.9 ± 0,9 |
| Heliobond®² | 1,0 ± 1,0 |
| Beispiel 2² | 2,1 ± 0,5 |

| | |
|---|---|
| ¹ Scherhaftfestigkeit nach ISO/TS 11405 ² Vergleich | |

Die Haftwerte des erfindungsgemäßen Materials liegen deutlich über denjenigen der Vergleichsmaterialien. Die Ergebnisse zeigen, daß mit den erfindungsgemäßen Werkstoffen ohne zusätzliche Säurebehandlung der Zahnoberfläche Haftwerte erzielt werden können, die denjenigen von Glasionomer-Zementen, die beipsielsweise zur Befestigung von Kronen eingesetzt werden, entsprechen.

### Beispiel 5: Bestimmung des Kontaktwinkels

Zur Ermittlung der Antihafteigenschaften der erfindungsgemäßen Werkstoffe wurde der Kontaktwinkel von destilliertem Wasser auf der Filmoberfläche mit einem Kontaktwinkelmeßgerät (Modell DAT 1100, Firma Fibro System AB, Schweden) gemessen. Dazu wurden zylinderförmige Probenkörper mit einem Durchmesser von 20 mm und einer Dicke von 2 mm hergestellt und für 2 x 3 min belichtet (Spectramat, Ivoclar Vivadent). Die nicht polymerisierte Schicht auf den Oberflächen der Probenkörper wurde mit Ethanol entfernt und anschließend an jedem Probenkörper 5 Messungen durchgeführt. Die Mittelwerte der Messungen sind in der nachfolgenden Tabelle 4 zusammengefaßt. Als Vergleich dienten handelsübliche dentale Beschichtungsmaterialien auf der Basis einer Methacrylatmischung (Luxatemp® Glaze & Bond, DMG, Hamburg; Heliobond®, Firma Ivoclar Vivadent) und ein Material gemäß Beispiel 2.

Wie aus Tabelle 4 deutlich wird, zeigen die Proben 1 bis 4 Kontaktwinkel, die deutlich über denjenigen der handelsüblichen Vergleichsmaterials liegen. Hieraus folgt, daß die Oberflächenenergie und damit die Gefahr der Plaquebildung für die erfindungsgemäßen Proben 1 bis 4 deutlich geringer als für diese Vergleichsmaterialien ist. Das Material gemäß Beispiel 2 ergibt zwar ebenfalls einen hohen Kontaktwinkel, weist jedoch, wie oben gezeigt wurde, eine deutlich schlechtere Haftung auf.

**Tabelle 4**

| **Kontaktwinkel für erfindungsgemäße Werkstoffe bestimmt mit destilliertem Wasser** | |
|---|---|
| **Werkstoff** | **Kontaktwinkel** |
| Nr. 1 | 94 |
| Nr. 2 | 90 |
| Nr. 3 | 94 |
| Nr. 4 | 90 |
| Luxatemp® Glaze & Bond¹ | 70 |
| Heliobond®¹ | 69 |
| Beispiel 2¹ | 93 |

| | |
|---|---|
| ¹ Vergleich | |

## Patentansprüche

1. Polymerisierbares dentales Beschichtungsmaterial , das
(1) 1 bis 80 Gew.-% mindestens eines Polykondensats auf der Basis von
(A) einem oder mehreren radikalisch polymerisierbaren Silanen der Formel (I)
(R¹ₐ-R²)ₘR³ₙSiX₍₄₋ₘ₋ₙ₎ (I),
in der R¹ eine radikalisch polymerisierbarer Gruppe ist, R² entfällt oder ein zwei- oder dreiwertiger, substituierter oder unsubstituierter Kohlenwasserstoffrest mit 1 bis 30 Kohlenstoffatomen ist, wobei die Kohlenstoffketten des Kohlenwasserstoffrestes durch ein oder mehrere Heteroatome, eine oder mehrere Carbonsäureestergruppen (-CO-O- oder -0-CO-), Carbonsäureamidgruppen (-CO-NR⁷- oder -NR⁷-CO- mit R⁷ = H oder C₁-C₅-Alkyl), Urethangruppen (-HN-CO-O- oder -O-CO-NH-) und/oder Aminogruppen (-NR⁸- mit R⁸ = H oder C₁-C₅-Alkyl) unterbrochen sein können, R³ ein C₁-C₁₂-Alkyl- oder C₃-C₁₂-Cycloalkylrest, C₆-C₁₂-Arylrest, C₆-C₁₂-Arylalkyl, C₆-C₁₂-Alkylaryl ist, X Halogen oder ein C₁-C₃-Alkoxyrest ist, a 1 oder 2 ist, m eine ganze Zahl von 1 bis 3 ist und n eine ganze Zahl von 0 bis 2 ist, wobei die Summe von m und n nicht größer als 3 ist sein kann, und gegebenenfalls
(B) einem oder mehreren Silanen gemäß der Formel (II)
R⁴₍₄₋ₚ₎SiYₚ (II),
in der R⁴ ein C₁-C₁₂-Alkylrest oder Phenylrest ist, die jeweils durch -F substituiert sein können, Y Halogen oder ein C₁-C₃-Alkoxyrest ist, und p eine ganze Zahl von 1 bis 4 ist, und/oder
(C) eine oder mehrere Metallverbindungen der Formeln (III) und/oder (IV)
AlZ₃ (III)
Me(O)ᵣZₛ (IV),
in denen Z Halogen oder ein C₁-C₆-Alkoxyrest ist, Me Zirkonium oder Titan ist, r 0 oder 1 ist, s eine ganze Zahl von 1 bis 4 ist, wobei die Summe der Wertigkeiten der Reste O und Z der Wertigkeit des Metalls entspricht,
(2) 0,1 bis 5 Gew.-% eines oder mehrerer Initiatoren für die radikalische Polymerisation und
(3) 0 bis 60 Gew.-% weiteres radikalisch polymerisierbares Monomer
enthält, **dadurch gekennzeichnet, daß** das Material außerdem
(4) 1 bis 50 Gew.-% eines oder mehrerer radikalisch polymerisierbarer Monomere der Formel (V),
R⁵ₜ-SP-A_{w} (V),
in der R⁵ eine radikalisch polymerisierbare Gruppe ist, t eine ganze Zahl von 1 bis 5 ist, Sp ein Kohlenwasserstoffrest mit 1 bis 30 Kohlenstoffatomen ist, wobei die Kohlenstoffketten des Kohlenwasserstoffrestes durch O oder S-Atome unterbrochen sein können, A -PO(OH)₂, -0-PO(OH)₂, -O-PO(OH)R⁶ oder -SO₃H ist, wobei R⁶ ein verzweigter oder unverzweigter C₁-C₂₀-Alkylrest, ein C₃-C₁₀-Cycloalkylrest oder ein aromatischer C₆-C₂₀-Rest ist, wobei R⁶ substituiert oder unsubstituiert sein kann, w eine ganze Zahl von 1 bis 3 ist;
enthält und das Polykondensat (1), das Monomer (3) oder beide durch Fluor substituiert sind, wobei das Material mindestens ein Polykondensat oder ein Monomer enthält, das durch Fluor substituiert ist.

2. Beschichtungsmaterial nach Anspruch 1, **dadurch gekennzeichnet, daß** das Polykondensat auf mindestens einem Silan der Formel (I) basiert, in dem mindestens eine Variable eine der folgenden Bedeutungen hat:
R¹ = Vinyl, (Meth)acryl, Vinylcyclopropyl, Allyl oder Styryl,
R² = entfällt, Methylen, Ethylen, Propylen, Butylen, Phenylen, - (CH₂)₂CH-O-OC-(CH₂)₃-CO-NR⁹-(CH₂)₃- oder-[CH₂-CHR⁹-O-OC-(CH₂)₂-]₂N-(CH₂)₃- mit R⁹ = H oder CH₃,
R³ = C₁-C₁₂-Alkyl, besonders bevorzugt Methyl oder Ethyl;
X = F, Cl oder C₁-C₃-Alkoxy, besonders bevorzugt F, Methoxy oder Ethoxy;
n = 0 oder 1
m = 1.

3. Beschichtungsmaterial nach einem der vorhergehenden Ansprüche, **dadurch gekennzeichnet, daß** das Polykondensat auf mindestens einem Silan der Formel (II) basiert, in dem mindestens eine Variable eine der folgenden Bedeutungen hat:
R⁴ = lineares C₁-C₁₂-Alkyl, insbesondere Methyl, Ethyl, Propyl, Phenyl, wobei die Wasserstoffatome von R⁴ ganz oder teilweise durch F ersetzt sind;
Y = F, Methoxy oder Ethoxy;
p = eine ganze Zahl von 1 bis 4.

4. Beschichtungsmaterial nach einem der vorhergehenden Ansprüche, **dadurch gekennzeichnet, daß** das Polykondensat auf mindestens einer Metallverbindung der Formeln (III) und/oder (IV) basiert, in denen Z C₁-C₄-Alkoxy oder Cl ist.

5. Beschichtungsmaterial nach einem der vorhergehenden Ansprüche, **dadurch gekennzeichnet, daß** es mindestens ein radikalisch polymerisierbares Monomer der Formel (V) enthält, in der mindestens eine der Variablen eine der folgenden Bedeutungen hat:
R⁵ = eine Vinyl-, (Meth)acryl-, (Meth)arylamid-, Styryl-Gruppe oder eine Gruppe der Formel (VI) 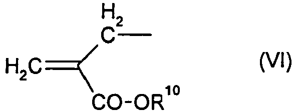 in der R¹⁰ H oder ein verzweigter oder vorzugsweise unverzweigter C₁-C₂₀-, vorzugsweise C₁-C₁₂-, insbesondere C₁-C₆-Alkylrest, ein C₃-C₂₀-, vorzugsweise C₃-C₆-Cycloalkyl, oder ein aromatischer C₆-C₂₀-, vorzugsweise C₆-C₁₀-Rest ist, wobei R¹⁰ substituiert oder unsubstituiert sein kann;
t = 1 oder 2;
Sp = C₁-C₁₅-Kohlenwasserstoffrest, der durch O-Atome, vorzugsweise 1 bis 5 O-Atome, unterbrochen sein kann;
A = -O-P(O)(OH)₂, -SO₃H, insbesondere -PO(OH)₂;
w = 1 oder 2.

6. Beschichtungsmaterial nach einem der vorhergehenden Ansprüche, **dadurch gekennzeichnet, daß** es zusätzlich (5) 0 bis 50 Gew.-% Füllstoff mit einer mittleren Partikelgröße von 5 bis 60 nm enthält.

7. Beschichtungsmaterial nach einem der vorhergehenden Ansprüche, **dadurch gekennzeichnet, daß** das Silan der Formel (II) durch Fluor substituiert ist.

8. Beschichtungsmaterial nach einem der vorhergehenden Ansprüche, **dadurch gekennzeichnet, daß** es mindestens ein weiteres radikalisch polymerisierbares Monomer (3) enthält, das bei Raumtemperatur flüssig ist und eine radikalisch polymerisierbare Gruppe aufweist.

9. Beschichtungsmaterial nach einem der vorhergehenden Ansprüche, **dadurch gekennzeichnet, daß** es mindestens ein weiteres radikalisch polymerisierbares Monomer (3) enthält, das zwei oder mehr radikalisch polymerisierbare Gruppen aufweist.

10. Beschichtungsmaterial nach einem der vorhergehenden Ansprüche, **dadurch gekennzeichnet, daß** es mindestens ein weiteres radikalisches Monomer (3) enthält, das durch Fluor substituiert ist.

11. Nicht-therapeutische Verwendung eines Beschichtungsmaterials nach einem der vorhergehenden Ansprüche zur Beschichtung von dentalen Prothesen oder orthodontischen Vorrichtungen.

12. Verwendung eines Beschichtungsmaterials nach einem der Ansprüche 1 bis 10 zur Herstellung eines Materials zur Beschichtung von Zähnen.

13. Verwendung nach Anspruch 12 zur Beschichtung von Zähnen durch
(i) Reinigen der zu behandelnde Oberfläche,
(ii) Aufbringen eines Beschichtungsmaterials gemäß einem der Ansprüche 1 bis 10; und
(iii)Härten des aufgebrachten Beschichtungsmaterials durch radikalische Polymerisation.

14. Verwendung eines Beschichtungsmaterials nach einem der Ansprüche 1 bis 10 zur Herstellung eines Mittels zur vorbeugenden Behandlung von Karies, Parodontitis, Gingivitis und/oder Hypersensibilitäten von Zähnen.

15. Nicht-therapeutisches Verfahren zur Beschichtung einer Substratoberfläche bei dem man
(i) die zu behandelnde Oberfläche reinigt,
(ii) ein Beschichtungsmaterial gemäß einem der Ansprüche 1 bis 10 aufbringt; und
(iii)man dann das aufgebrachte Beschichtungsmaterial durch radikalische Polymerisation härtet.

16. Verfahren nach Anspruch 15, **dadurch gekennzeichnet, daß** man im Anschluß an Schritt (iii) eine Inhibierungsschicht entfernt.

17. Verfahren nach Anspruch 15 oder 16, **dadurch gekennzeichnet, daß** es keine Säurebehandlung der Substratoberfläche umfaßt.

18. Verfahren nach einem der Ansprüche 15 bis 17 zur kosmetischen Behandlung von natürlichen oder künstlichen Zähnen.

19. Kit enthaltend ein Beschichtungsmaterial gemäß einem der Ansprüche 1 bis 10, wobei das Beschichtungsmaterial in einem luft- und lichtdichten Behälter untergebracht ist.

20. Kit nach Anspruch 19, weiter enthaltend eine Applikationshilfe für den Werkstoff.

## Claims

1. Polymerisable dental coating material that comprises
(1) 1 to 80 wt % of at least one polycondensate based on
(A) one or more radically polymerisable silanes of Formula (I)
(R¹ₐ-R²)ₘR³ₙSiX₍₄₋ₘ₋ₙ₎ (I)
in which R¹ is a radically polymerisable group, R² is absent or is a di or trivalent, substituted or unsubstituted hydrocarbon residue with 1 to 30 carbon atoms, wherein the carbon chains of the hydrocarbon residue may be interrupted by one or more heteroatoms, one or more carboxylic acid ester groups (-CO-O- or -O-CO-), carboxylic acid amide groups (-CO-NR⁷- or -NR⁷-CO- with R⁷ = H or C₁-C₅ alkyl), urethane groups (-HN-CO-O- or -O-CO-NH-) and/or amino groups (-NR⁸- with R⁸ = H or C₁-C₅ alkyl), R³ is a C₁-C₁₂ alkyl or C₃-C₁₂ cycloalkyl residue, C₆-C₁₂ aryl residue, C₆-C₁₂ arylalkyl, C₆-C₁₂ alkylaryl, X is halogen or a C₁-C₃ alkoxy residue, a is 1 or 2, m is an integer from 1 to 3 and n is an integer from 1 to 2, wherein the sum of m and n may not be greater than 3, and optionally
(B) one or more silanes of Formula (II)
R⁴₍₄₋ₚ₎SiYₚ (II),
in which R⁴ is a C₁-C₁₂ alkyl residue or phenyl residue, each of which may be substituted by -F, Y is halogen or a C₁-C₃ alkoxy residue, and p is an integer from 1 to 4, and/or
(C) one or more metal compounds of Formulae (III) and/or (IV)
AlZ₃ (III)
Me(O)ᵣZₛ (IV),
in which Z is halogen or a C₁-C₃ alkoxy residue, Me is zirconium or titanium, r is 0 or 1, s is an integer from 1 to 4, wherein the sum of the valencies of the residues O and Z corresponds to the valency of the metal,
(2) 0.1 to 5 wt % of one or more initiators for the radical polymerisation and
(3) 0 to 60 wt % of another radically polymerisable monomer **characterised in that** the material further comprises
(4) 1 to 50 wt % of one or more radically polymerisable monomers of Formula (V),
R⁵ₜ-Sₚ-A_{w} (V),
in which R⁵ is a radically polymerisable group, t is an integer from 1 to 5, Sp is a hydrocarbon residue with 1 to 30 carbon atoms, wherein the carbon chains of the hydrocarbon residues may be interrupted by O or S atoms, A is -PO(OH)₂, -O-PO(OH)₂, -O-PO(OH)R⁶ or -SO₃H, wherein R⁶ is a branched or unbranched C₁-C₂₀ alkyl residue, a C₃-C₁₀ cycloalkyl residue or an aromatic C₆-C₂₀ residue, wherein R⁶ may be substituted or unsubstituted, w is an integer from 1 to 3;
and the polycondensate (1), the monomer (3) or both are substituted by fluorine, wherein the material comprises at least one polycondensate or one monomer, which is substituted by fluorine.

2. Coating material according to claim 1, **characterised in that** the polycondensate is based on at least one silane of Formula (I), in which at least one variable has one of the following meanings:
R¹ = vinyl, (meth)acryl, vinylcyclopropyl, allyl or styryl,
R² = absent, methylene, ethylene, propylene, butylene, phenylene,-(CH₂)₂CH-O-OC-(CH₂)₃-CO-NR⁹-(CH₂)₃ or -[CH₂CHR⁹-O-OC-(CH₂)₂-]₂N-(CH₂)₃ with R⁹ = H or CH₃,
R³ = C₁-C₁₂ alkyl, particularly preferably methyl or ethyl;
X = F, Cl or C₁-C₃ alkoxy, particularly preferably F, methoxy or ethoxy;
n = 0 or 1
m=1.

3. Coating material according to one of the preceding claims, **characterised in that** the polycondensate is based on at least one silane of Formula (II), in which at least one variable has one of the following meanings:
R⁴ = linear C₁-C₁₂ alkyl, in particular methyl, ethyl, propyl, phenyl, wherein the hydrogen atoms of R⁴ are completely or partially replaced by F;
Y = F, methoxy or ethoxy;
p = a whole number from 1 to 4.

4. Coating material according to one of the preceding claims, **characterised in that** the polycondensate is based on at least one metal compound of Formulae (III) and/or (IV), in which Z is C₁-C₄ alkoxy or Cl.

5. Coating material according to one of the preceding claims, **characterised in that** it comprises at least one radically polymerisable monomer of Formula (V), in which at least one of the variables has one of the following meanings:
R⁵ = a vinyl, (meth)acryl, (meth)arylamide, styryl group or a group of Formula (VI) 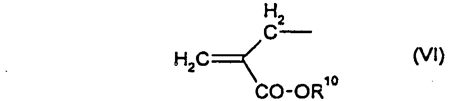 in which R¹⁰ is H or a branched or preferably unbranched C₁-C₂₀, preferably C₁-C₁₂, in particular C₁-C₆ alkyl residue, a C₃-C₂₀, preferably C₃-C₆ cycloalkyl, or an aromatic C₆-C₂₀, preferably C₃-C₁₀ residue, wherein R¹⁰ may be substituted or unsubstituted;
t= 1 or 2;
Sp = C₁-C₁₅ hydrocarbon residue that may be interrupted by O atoms, preferably 1 to 5 O atoms;
A = -O-P(O)(OH)₂, -SO₃H, in particular -PO(OH)₂;
w = 1 or 2.

6. Coating material according to one of the preceding claims, **characterised in that** it
(5) additionally comprises 0 to 50 wt % filler with an average particle size of 5 to 60 nm.

7. Coating material according to one of the preceding claims, **characterised in that** the silane of Formula (II) is substituted by fluorine.

8. Coating material according to one of the preceding claims, **characterised in that** it comprises at least one further radically polymerisable monomer (3) that is liquid at room temperature and possesses a radically polymerisable group.

9. Coating material according to one of the preceding claims, **characterised in that** it comprises at least one further radically polymerisable monomer (3) that possesses two or more radically polymerisable groups.

10. Coating material according to one of the preceding claims, **characterised in that** it comprises at least one further radically polymerisable monomer (3) that is substituted by fluorine.

11. Non-therapeutic use of a coating material according to one of the preceding claims for coating dental prostheses or orthodontic devices.

12. Use of a coating material according to one of claims 1 to 10 for producing a material for coating teeth.

13. Use according to claim 12 for coating teeth by
(i) cleaning the surface to be treated,
(ii) depositing a coating material according to one of claims 1 to 10; and
(iii) curing the deposited coating material by radical polymerisation.

14. Use of a coating material according to one of claims 1 to 10 for producing a means for the preventative treatment of caries, paeriodontitis, gingivitis and/or hypersensitivities of teeth.

15. Non-therapeutic method for coating a substrate surface in which method
(i) the surface to be treated is cleaned,
(ii) a coating material according to one of claims 1 to 10 is deposited; and
(iii) the deposited coating material is cured by radical polymerisation.

16. Method according to claim 15, **characterised in that** subsequent to step (iii) an inhibition layer is removed.

17. Method according to claim 15 or 16, **characterised in that** it does not include an acidic treatment of the substrate surface.

18. Method according to one of claims 15 to 17 for the cosmetic treatment of natural or synthetic teeth.

19. Kit comprising a coating material according to one of claims 1 to 10, wherein the coating material is housed in an air- and light-tight container.

20. Kit according to claim 19, further comprising an application aid for the material.

## Revendications

1. Matériau de revêtement dentaire polymérisable, qui contient
(1) 1 à 80 % en poids d'au moins un produit de polycondensation à base de
(A) un ou plusieurs silanes aptes à la polymérisation radicalaire, de formule (I)
(R¹ₐR²)ₘR³ₙSiX₍₄₋ₘ₋ₙ₎ (I),
dans laquelle R¹ est un groupe apte à la polymérisation radicalaire, R² est absent ou est un radical hydrocarboné mono- ou divalent, substitué ou non substitué, ayant de 1 à 30 atomes de carbone, les chaînes carbonées du radical hydrocarboné pouvant être interrompues par un ou plusieurs hétéroatomes, un ou plusieurs groupes ester d'acide carboxylique (-CO-O- ou -O-CO-), groupes carboxamido (-CO-NR⁷-ou -NR⁷-CO- où R⁷ = H ou un groupe alkyle en C₁-C₅), groupes uréthane (-NH-CO-O- ou -O-CO-NH-) et/ou groupes amino (-NR⁸- où R⁸ = H ou un groupe alkyle en C₁-C₅), R³ est un radical alkyle en C₁-C₁₂ ou cycloalkyle en C₃-C₁₂, un radical aryle en C₆-C₁₂, arylalkyle en C₆-C₁₂, alkylaryle en C₆-C₁₂, X est un atome d'halogène ou un radical alcoxy en C₁-C₃, a est 1 ou 2, m est un nombre entier valant de 1 à 3 et n est un nombre entier valant de 0 à 2, la somme de m et n ne pouvant excéder 3, et éventuellement
(B) un ou plusieurs silanes selon la formule (II)
R⁴₍₄₋ₚ₎SiYₚ (II),
dans laquelle R⁴ est un radical alkyle en C₁-C₁₂ ou un radical phényle, qui peuvent être substitués chacun par -F, Y est un atome d'halogène ou un radical alcoxy en C₁-C₃, et p est un nombre entier valant de 1 à 4, et/ou
(C) un ou plusieurs composés métalliques de formules (III) et/ou (IV)
AlZ₃ (III) Me(O)ᵣZₛ (IV),
dans lesquelles Z est un atome d'halogène ou un radical alcoxy en C₁-C₆, Me est un atome de zirconium ou de titane, r est 0 ou 1, s est un nombre entier valant de 1 à 4, la somme des valences des radicaux O et Z correspondant à la valence du métal,
(2) 0,1 à 5 % en poids d'un ou de plusieurs amorceurs pour la polymérisation radicalaire et
(3) 0 à 60 % en poids d'un autre monomère apte à la polymérisation radicalaire, **caractérisé en ce que** le matériau contient en outre
(4) 1 à 50 % en poids d'un ou de plusieurs monomères aptes à la polymérisation radicalaire, de formule (V),
R⁵ₜ-Sp-Aw (V),
dans laquelle R⁵ est un groupe apte à la polymérisation radicalaire, t est un nombre entier valant de 1 à 5, Sp est un radical hydrocarboné ayant de 1 à 30 atomes de carbone, les chaînes carbonées du radical hydrocarboné pouvant être interrompues par des atomes d'oxygène ou de soufre, A est -PO(OH)₂, -O-PO(OH)₂, -O-PO(OH)R⁶ ou -SO₃H, R⁶ étant un radical alkyle en C₁-C₂₀ ramifié ou non ramifié, un radical cycloalkyle en C₃-C₁₀ ou un radical aromatique en C₆-C₂₀, R⁶ pouvant être substitué ou non substitué, w est un nombre entier valant de 1 à 3 ;
et le produit de polycondensation (1) le monomère (3) ou les deux sont substitués par le fluor, le matériau contenant au moins un produit de polycondensation ou un monomère qui est substitué par le fluor.

2. Matériau de revêtement selon la revendication 1, **caractérisé en ce que** le produit de polycondensation est à base d'au moins un silane de formule (I), dans lequel au moins une variable a l'une des significations suivantes :
R¹ = vinyle, (méth)acryloyle, vinylcyclopropyle, allyle ou styryle,
R² est absent ou représente un groupe méthylène, éthylène, propylène, butylène, phénylène, -(CH₂)₂CH-O-OC-(CH₂)₃-CO-NR⁹-(CH₂)₃- ou -[CH₂-CHR⁹-O-OC-(CH₂)₂-]₂N-(CH₂)₃- où R⁹ = H ou CH₃,
R³ = alkyle en C₁-C₁₂, de façon particulièrement préférée méthyle ou éthyle ;
X = F, Cl ou alcoxy en C₁-C₃, de façon particulièrement préférée F, méthoxy ou éthoxy ;
n = 0 ou 1
m = 1.

3. Matériau de revêtement selon l'une quelconque des revendications précédentes, **caractérisé en ce que** le produit de polycondensation est à base d'au moins un silane de formule (II), dans lequel au moins une variable a l'une de significations suivantes :
R⁴ = un groupe alkyle en C₁-C₁₂ linéaire, en particulier méthyle, éthyle, propyle, phényle, les atomes d'hydrogène de R⁴ étant en partie ou en totalité remplacés parF;
Y F, méthoxy ou éthoxy ;
p = un nombre entier valant de 1 à 4.

4. Matériau de revêtement selon l'une quelconque des revendications précédentes, **caractérisé en ce que** le produit de polycondensation est à base d'au moins un composé métallique de formules (III) et/ou (IV), dans lesquelles Z est un groupe alcoxy en C₁-C₄ ou Cl.

5. Matériau de revêtement selon l'une quelconque des revendications précédentes, **caractérisé en ce qu'**il contient au moins un monomère apte à la polymérisation radicalaire, de formule (V), dans laquelle au moins l'une des variables a l'une de significations suivantes :
R⁵ est un groupe vinyle, (méth)acryloyle, (méth)acrylamide, styryle ou un groupe de formule (VI) 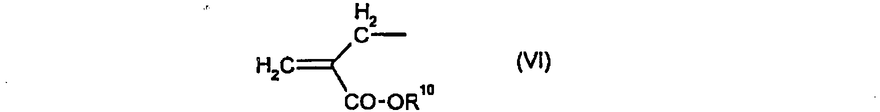 dans laquelle R¹⁰ est H ou un radical alkyle en C₁-C₂₀, de préférence en C₁-C₁₂, en particulier en C₁-C₆, ramifié ou de préférence non ramifié, un radical cycloalkyle en C₃-C₂₀, de préférence en C₃-C₆, ou un radical aromatique en C₆-C₂₀, de préférence en C₆-C₁₀, R¹⁰ pouvant être substitué ou non substitué ;
t = 1 ou 2 ;
Sp = un radical hydrocarboné en C₁-C₁₅ qui peut être interrompu par des atomes d'oxygène, de préférence 1 à 5 atomes d'oxygène ;
A = -O-P(O)(OH)₂, -SO₃H, en particulier -PO(OH)₂ ;
w = 1 ou 2.

6. Matériau de revêtement selon l'une quelconque des revendications précédentes, **caractérisé en ce qu'**il contient en outre
(5) 0 à 50 % en poids de charge ayant une taille moyenne de particule de 5 à 60 nm.

7. Matériau de revêtement selon l'une quelconque des revendications précédentes, **caractérisé en ce que** le silane de formule (II) est substitué par le fluor.

8. Matériau de revêtement selon l'une quelconque des revendications précédentes, **caractérisé en ce qu'**il contient au moins un autre monomère (3) apte à la polymérisation radicalaire, qui est liquide à la température ambiante et comporte un groupe apte à la polymérisation radicalaire.

9. Matériau de revêtement selon l'une quelconque des revendications précédentes, **caractérisé en ce qu'**il contient au moins un autre monomère (3) apte à la polymérisation radicalaire, qui comporte deux ou plus de deux groupes aptes à la polymérisation radicalaire.

10. Matériau de revêtement selon l'une quelconque des revendications précédentes, **caractérisé en ce qu'**il contient au moins un autre monomère (3) apte à la polymérisation radicalaire, qui est substitué par le fluor.

11. Utilisation non thérapeutique d'un matériau de revêtement selon l'une quelconque des revendications précédentes, pour le revêtement de prothèses dentaires ou de dispositifs orthodontiques.

12. Utilisation d'un matériau de revêtement selon l'une quelconque des revendications 1 à 10, pour la production d'un matériau destiné au revêtement de dents.

13. Utilisation selon la revendication 12, pour le revêtement de dents par
(i) nettoyage de la surface à traiter,
(ii) application d'un matériau de revêtement selon l'une quelconque des revendications 1 à 10 ; et
(iii) durcissement par polymérisation radicalaire du matériau de revêtement appliqué.

14. Utilisation d'un matériau de revêtement selon l'une quelconque des revendications 1 à 10, pour la production d'une composition destinée au traitement préventif de caries, parodontose, gingivite et/ou d'hypersensibilités des dents.

15. Procédé non thérapeutique destiné au revêtement d'une surface de substrat, dans lequel
(i) on nettoie la surface à traiter,
(ii) on applique un matériau de revêtement selon l'une quelconque des revendications 1 à 10 ; et
(iii) on fait durcir par polymérisation radicalaire le matériau de revêtement appliqué.

16. Procédé selon la revendication 15, **caractérisé en ce qu'**à la suite de l'étape (iii) on élimine une couche d'inhibition.

17. Procédé selon la revendication 15 ou 16, **caractérisé en ce qu'**il ne comprend aucun traitement par un acide de la surface du substrat.

18. Procédé selon l'une quelconque des revendications 15 à 17, pour le traitement cosmétique de dents naturelles ou artificielles.

19. Nécessaire contenant un matériau de revêtement selon l'une quelconque des revendications 1 à 10, le matériau de revêtement étant placé dans un contenant étanche à l'air et opaque.

20. Nécessaire selon la revendication 19, contenant en outre un dispositif d'application, pour le matériau.
